# EUROPEAN PATENT APPLICATION

(11) **EP 3 714 919 A1**
(43) Date of publication of application: **30.09.2020**
(21) Application number: 19166129.7
(22) Date of filing: 29.03.2019
(51) Int. Cl.: A61M 1/36, F16K 15/00, A61M 39/24

(54) **REINFUSION TUBE SYSTEM, PACKAGE AND METHODS**

(71) Applicant: Fresenius Medical Care Deutschland GmbH, 61352 Bad Homburg (DE)
(72) Inventor: Häcker, Jürgen, 61267 Neu-Anspach (DE); Manke, Joachim, 35792 Löhnberg (DE); Müller, Ralf, 61350 Bad Homburg (DE); Davico, Edoardo, 26013 Crema (IT)
(74) Representative: Bobbert & Partner Patentanwälte PartmbB

(57) **Abstract**

The present invention relates to a reinfusion tube system (10) comprising a reinfusion fluid tubing comprising a pre-pump loop section (20), a post-pump loop section (30), and a pump loop section (40); and one of
- a check valve (70) positioned at an end or between the ends of the reinfusion fluid tubing such that it is provided to block fluid flow through the check valve (70) in a direction from the post-pump loop section (30) to the pre-pump loop section (20), and
- a check valve adapter (I, II, III) comprising a check valve (70), the check valve adapter (I, II, III) being configured to be connected to an end or to be positioned between the ends of the reinfusion fluid tubing such that the check valve (70) is provided to block fluid flow through the check valve (70) in a direction from the post-pump loop section (30) to the pre-pump loop section (20).

## Description

### FIELD OF THE INVENTION

The present invention relates to a reinfusion tube system according to the preamble of claim 1, a reinfusion tube system package according to the preamble of claim 13 and a system according to the preamble of claim 15. The present invention further relates to a method according to claim 16, a method according to claim 17 as well as to a check valve adapter according to claim 20.

### BACKGROUND OF THE INVENTION

In practice, for treating patients' blood outside the body using extracorporeal blood treatment devices, for example, dialysis, hemodialysis, hemodiafiltration, ultrafiltration, plasmapherese devices, tubing sets or similar disposables are used, through which the blood flows during the treatment. For example, during dialysis or ultrafiltration treatment, blood is guided through an arterial blood line, a venous blood line and a dialyzer which are brought into fluid connection with each other. The arterial blood line and the venous blood line are connected to the patient's vascular system with one of their ends, respectively, for example via a needle or a catheter, and the arterial blood line is used as supply line to guide blood to the dialyzer and the venous line is used as a return line to guide blood from the dialyzer back to the patient. Together, the arterial and the venous blood lines are called "blood tubing set" or simply "tubing set". The tubing set may also comprise further components.

After the treatment, the tubing set and the dialyzer are full of blood which shall be returned ("reinfused") to the patient before disconnecting the patient from the blood tubing set.

Regularly, for the purpose of reinfusion liquid is guided via a reinfusion fluid tubing into the blood tubing set and towards the dialyzer in order to push, using the liquid, the blood back into the patient. Thereby, the blood in the extracorporeal blood circuit is at least to a large extent replaced by the reinfusion liquid. This step is regularly called "reinfusion of the blood"; another expression is, for example, "blood return".

Before the treatment and before having connected the patient's vascular system to the tubing set the tubing set is regularly filled with a physiological solution, also called priming liquid. This step is regularly called "priming of the blood tubing set".

Regarding a set configuration for the liquid supply, the reinfusion step may be similar to the priming step. However, during priming the venous line may be connected to a drain port of the dialysis device, whereas during the reinfusion process the venous line is still connected to the patient. So, when the expression "reinfusion solution" or "reinfusion liquid" is used hereinafter, this also encompasses the priming liquid meaning the liquid used for initially filling the tubing set.

There are several methods known to move or convey liquid present in the blood tubing set by using a physiological solution in order to reinfuse the blood. For example, liquid out of a bag containing physiological solution or dialysis fluid provided by a fluid system of the extracorporeal blood treatment device may be used. This dialysis fluid is also often called "replacement liquid" or "reinfusion liquid".

For the conventional reinfusion method, a reinfusion fluid tubing connects the reinfusion liquid source to the blood tubing set. By pumping the reinfusion solution from the reinfusion liquid source to the blood tubing set the blood is pushed out of the tubing set. A peristaltic pump may be used to pump the reinfusion liquid and, to this end, the reinfusion fluid tubing may comprise a pump loop segment which in turn comprises a flexible tubing element that may be squeezed for pumping purposes. For reinfusing the blood, both the arterial line and the venous line may remain connected to the dialyzer with one of their ends, whereas the reinfusion fluid tubing is connected to the patient end (the second end opposite to the dialyzer connected end) of the arterial line.

In this configuration, blood in the arterial line, the dialyzer and the venous line may be pushed towards the venous patient end and out of the tubing set to be replaced by reinfusion liquid.

In particular, in the so-called in-center treatment one patient after the other is treated using the same extracorporeal blood treatment device. For avoiding cross-contamination from one patient to the next all elements through which blood is intended to flow are normally not re-used for the consecutive patient.

Other elements, however, that are also in fluid connection with, during the treatment, blood containing parts are not replaced for each treatment or from patient to patient. These re-used elements are for example the dialysis fluid carrying hoses or the tubing of the so-called hydraulics of the extracorporeal blood treatment device, and/or the hoses or the tubing guiding the dialysis fluid as reinfusion liquid to a reinfusion port to which the reinfusion fluid tubing may be connected for reinfusion. The fluid connection to the blood guiding parts may be established for example via a dialyzer membrane, via the priming line, or via the reinfusion fluid tubing to the blood tubing system. These re-used elements are, under normal conditions and during the conventional use, not intended to come into direct contact with the blood, and therefore, measures are needed to ensure this, so that there is no risk of cross-contamination. Such measures are, for example, that the system is regularly disinfected, that the system is run safely, e.g. that it is ensured that at any time the pressure gradient ensures that at critical positions no flow of blood towards the re-used parts occurs, that occluding pumps are used or that blood leak detection systems are used in the re-used parts to recognize blood loss into the re-used parts.

### SUMMARY OF THE INVENTION

The objective of the present invention may be achieved by the reinfusion tube system having the features of claim 1. It may further be achieved by the reinfusion tube system package having the features of claim 13 and the system having the features of claim 15 as well as by the method having the features of claim 16 and the method having the features of claim 17, moreover by the check valve adapter having the features of claim 20.

In all of the aforementioned and following statements, the use of the expression "may be" or "may have" and so on, is to be understood synonymously with "preferably is" or "preferably has", and so on respectively, and is intended to illustrate an embodiment according to the present invention.

Whenever numerical words are mentioned herein, the person skilled in the art shall recognize or understand them as indications of numerical lower limits. Hence, unless this leads to a contradiction evident for the person skilled in the art, the person skilled in the art shall comprehend for example "one" as encompassing "at least one". This understanding is also equally encompassed by the present invention as the interpretation that a numerical word, for example, "one" may alternatively mean "exactly one", wherever this is evidently technically possible in the view of the person skilled in the art. Both of these understandings are encompassed by the present invention and apply herein to all used numerical words.

The reinfusion tube system comprises a reinfusion fluid tubing having or consisting of at least three sections of a pre-pump loop section, a post-pump loop section, and a pump loop section, or consists thereof. The pump loop section may be positioned with respect to a fluid flow through the reinfusion fluid tubing between the pre-pump loop section and the post-pump loop section and optionally may be in contact with them.

Further, the reinfusion tube system comprises a check valve, and the check valve is configured to be positioned at an end or between the ends of the fluid tubing and the check valve has a blocking orientation to block the flow in a direction from the post-pump loop section to the pre-pump loop section.

Alternatively or in addition, the reinfusion tube system according to the present invention comprises a check valve adapter comprising in turn a check valve. Herein, the check valve adapter is optionally designed or configured to be connected to an end, or to be positioned between the ends, of the reinfusion fluid tubing such that the check valve mentioned supra is provided to block fluid through the check valve in a direction from the post-pump loop section to the pre-pump loop section.

The expression "tubing" has the meaning of an at least partially flexible tube or hose or, in other words, the tubing may be bent. This distinguishes the "tubing" from, for example, hard plastic channels. For example, one or more of the sections, in particular the pre-pump loop section and/or the post-pump loop section may comprise such flexible or bendable tubing. For connecting the sections to each other one or more connectors may be provided between the sections. The connectors may be dimensionally stable with respect to a fluid path through the connector.

The expressions "pre-pump loop" and "post-pump loop" refer to the direction when a liquid is supplied from the dialysis device towards the blood tubing set. Thus, "pre-pump loop" corresponds to an upstream position and "post-pump loop" corresponds to a downstream position relative to the pump-loop section when a liquid is supplied from the extracorporeal blood treatment device towards the blood tubing set, in particular, the arterial blood line.

In other words, the check valve may be positioned at any point along the reinfusion fluid tubing, also at an end of the reinfusion fluid tubing, in particular as long as it blocks a flow of liquid entering into the post-pump loop section from exiting via the pre-pump loop section of the reinfusion tube system into the hydraulics or the fluid system of the extracorporeal blood treatment device when being used.

According to the present invention, a method for reinfusing blood in a blood tubing set encompasses firstly the step of connecting the reinfusion fluid tubing to both a reinfusion port of an extracorporeal blood treatment device and to an arterial line. The method encompasses secondly the step of supplying a reinfusion fluid from a reinfusion fluid source via the reinfusion port to the reinfusion tube system.

According to the present invention, a method for preparing or setting up an extracorporeal blood treatment device with a reinfusion fluid tubing before reinfusing blood into a patient's vessel encompasses the following steps:
a) Connecting the reinfusion tube system according to the present invention to a reinfusion port of the extracorporeal blood treatment device, such that the check valve is positioned or orientated so as to block fluid flow through it, in particular in a direction from the post-pump loop section to the pre-pump loop section and/or towards the extracorporeal blood treatment device;
b) or connecting the check valve adapter of the reinfusion tube system to the reinfusion fluid tubing, in particular to the post-pump loop section.

A check valve adapter according to the present invention comprises or consists of a first tubular element and a second tubular element which is in fluid communication with the first tubular element. A check valve is arranged in this fluid communication.

The reinfusion fluid tubing may also comprise one or two line connectors at one or two of its ends, respectively, wherein the line connectors may be irremovably or removably attached or attachable to these ends.

The expression "section" does not necessarily have the meaning of being physically separate elements. This may be the case, but the sections may also be in any combination formed as integrally built or designed elements. "Section" may also encompass the line connector or the line connectors provided to connect the sections.

The expression "positioned" has the meaning of defining the location of the check valve in or at one of the sections of the reinfusion tube system.

The check valve may be in fluid connection with the inner lumen of the tubes or sections of the reinfusion tube system.

Any one of the pre-pump loop section, the post-pump loop section and/or the pump loop section may be formed by one or more separate tubing elements. In particular, when it is described that the check valve may be positioned in or on a section this may mean two tubing elements forming a section with the check valve in-between or in a connector connecting the two tubing elements with each other. The same applies to line connectors or connectors between the sections.

Further developments of the present invention are each subject-matter of the dependent claims and embodiments.

Whenever an embodiment is mentioned herein, it represents an exemplary embodiment according to the present invention.

Embodiments according to the present invention may comprise one or several of the features mentioned supra and/or in the following in any combination which is technically possible.

In some embodiments, the reinfusion tube system is not part of a cassette system. A cassette may be understood as a complex and highly integrated assembly. For example, it may have a, or one, hard body or another physical body comprising channels to guide liquids. It may also have at least one further element of the group consisting of a measuring interface to measure a pressure within the cassette, an expansion chamber for liquid, a gas separation chamber to separate gas from blood, and a solution supplying pump loop connected to the channel intended for guiding blood.

The pre-pump loop section and the post-pump loop section may be constructed differently from the loop in the pump loop section as, e.g., the material of this loop may be optimized. The pump loop section may have, for example, one or more tubes of a higher flexibility, larger diameter, and/or higher stability, for the pumping action of a peristaltic pump.

An end of the reinfusion tube system may comprise a female Luer lock to be connected with a male Luer lock arranged, for example, at an end of an arterial blood line. The post-pump loop section may have a male Luer lock end and a check valve adapter, functioning as a transition piece, having, for example, two ends with female Luer lock connections. The check valve adapter may be part of the reinfusion tube system.

For connecting the post-pump loop section to the arterial line the check valve adapter or the transition piece may be attached as part of the post-pump loop section between to the male end of the post-pump loop section and the male part of the arterial blood line. This may cause the reinfusion tube system to correspond to the standard needle connection design and, accordingly, may fit to the end of the arterial line without further measures or connectors.

In other words, the reinfusion tube system may further comprise a line connector attached or attachable to a part forming an end of the post-pump loop section. This line connector may be configured to change the gender of the connector at the end of the post-pump loop section. The check valve may also be integrated in the transition piece or the check valve adapter changing or adapting the gender of the line connector at the end of the post-pump loop section.

In some embodiments of the reinfusion tube system according to the present invention, the check valve adapter comprises one first tubular element and one second tubular element and the check valve.

In several embodiments the reinfusion tube system according to the present invention further comprises a port connector having a first end and a second end. The first end is configured for being connected to a reinfusion liquid port of an extracorporeal blood treatment device, whereas the second end is to be attached or configured to be attached to the pre-pump loop section. In this, the port connector provides a fluid connection from the reinfusion liquid port to the pre-pump loop section.

In some embodiments the attaching may be via an element comprising the check valve and/or the check valve may be integrated in the port connector to be connected to the reinfusion liquid port.

In several embodiments of the reinfusion tube system according to the present invention the check valve adapter is configured to be connected to an arterial blood line with a first end for providing a fluid connection from the post-pump loop section to the arterial line.

The check valve may be arranged in a housing. The housing may be a hard plastic or resin or hard resin part, a hose element or a tube element. The housing may be non-removably attached or removably attachable to at least one of the three sections, in particular the pre-pump loop section or the post-pump loop section and/or the check valve adapter or between any two of these.

In some embodiments of the reinfusion tube system according to the present invention, the check valve is arranged in the port connector or line connector.

In several embodiments of the reinfusion tube system according to the present invention, the check valve is arranged in the connector used to connect the pre-pump loop section and/or the post-pump loop section to the pump-loop section.

In other words, the check valve may be integrated into a separate housing element which is or may be brought into fluid connection with the tube elements of the reinfusion tube system. The check valve may also be directly integrated into a tube of the reinfusion tube system.

In some embodiments the reinfusion tube system according to the present invention further comprises an arterial blood line and/or a venous blood line.

The check valve may have a blocking characteristic so that in a normal configuration it is closed. The expression "normal" has the meaning of a state where on both sides of the check valve the same pressure exists, in particular if on both sides ambient pressure exists. This means that a minimum pressure is required to open the check valve. The minimum opening pressure may be 200 mbar, 300 mbar, 400 mbar or 500 mbar, preferably 300 mbar. Due to the orientation of the check valve to allow a flow from the pre-pump loop section to the post-pump loop section such an opening pressure in a forward direction allows flow of liquid in the direction towards an arterial line when attached. In the opposite direction, which corresponds to the closing or blocking direction of the check valve, the maximum backpressure at which the check valve still blocks a flow may be 6.9 bar, 6 bar, 5 bar, 4 bar, 2.8 bar, 2.7 bar or 2.5 bar. The backpressure is the differential pressure between the outlet and inlet pressures at the opposite ends of the check valve in a closing direction. "Outlet" and "inlet" refer to the direction in which fluid is, under normal conditions, intended to flow through the check valve. The opening pressure corresponds to the inlet pressure at which the first indication of flow occurs. The opening pressure is also called cracking pressure.

The check valve may comprise a flow-through opening and a movable element, in particular a flexible element, wherein the movable element closes the flow-through opening in the blocking direction. It is understood that also in the opposite direction a flow may be blocked at low pressure differences across the check valve, but in the inlet-to-outlet direction the check valve opens the flow-though opening at a lower pressure difference than in the blocking direction. The check valve may comprise a stable form housing. Upon liquid pressure from one side, the flexible element may be deformed so that a fluid may flow through the housing. Upon liquid pressure from the opposite side, such deformation opening the fluid connection may not occur at least up to a pressure at which the check valve opens from the one side. Preferably, the ratio between the opening pressure and the backpressure is or is below 0.2, 0.1, or 0.05. The movable element may also be a non-flexible element and may be moved between two positions, one in which the flow-through opening is open and one in which the flow-through opening is closed. Such a movable, non-flexible or flexible element may be for example a ball or a piston.

The numbers provided above for the parameters - minimum opening pressure, the maximum backpressure, the ratio - may be present only by itself or in any combination of two of the parameters. For example the minimum opening pressure may be 300 mbar in combination with a maximum backpressure of 2.8 bar, in another example the minimum opening pressure may be 300 mbar in combination with a maximum backpressure of 6.9 bar, in another example the minimum opening pressure may be 400 mbar in combination with a maximum backpressure of 2.8 bar, in another example the minimum opening pressure may be 400 mbar in combination with a maximum backpressure of 6.9 bar. In another example the minimum opening pressure may be 300 mbar in combination with a ratio of 0.1, in another example the minimum opening pressure may be 300 mbar in combination with a ratio of 0.05, in another example the minimum opening pressure may be 400 mbar in combination with a ratio of 0.1, in another example the minimum opening pressure may be 400 mbar in combination with a ratio of 0.05, in another example the minimum opening pressure may be 500 mbar in combination with a ratio of 0.1, in another example the minimum opening pressure may be 500 mbar in combination with a ratio of 0.05, in another example the backpressure may be 6.9 bar in combination with a ratio of 0.1, in another example the backpressure may be 6.9 bar in combination with a ratio of 0.05, in another example the backpressure may be 2.8 bar in combination with a ratio of 0.1, in another example the backpressure may be 2.8 bar in combination with a ratio of 0.05, in another example the backpressure may be above 2.8 bar in combination with a ratio of 0.2.

The flexible element material may be made of an elastic polymer, e.g. silicone, and the housing may be made of a polycarbonate or comprise the same.

Further, a reinfusion tube system package may be provided wherein the package may comprise the reinfusion tube system, an arterial blood line and/or a venous blood line, wherein the reinfusion tube system, the arterial blood line and/or the venous blood line are packaged in a system container. The system container may be a bag or a blister pack or a solid container. The bag may be made of a plastic material; the blister pack may comprise two sheets of different materials one sheet attached to each other each forming a side of the bag.

In some embodiments the reinfusion tube system package according to the present invention comprises further an element container, in particular a bag or a blister pack, within which the check valve adapter is comprised. Moreover, the element container may be comprised within the system container.

The different materials may be a plastic material and cellulose based material, respectively. The plastic material may be transparent and the paper material may be non-transparent or opaque. Printing may be provided on the bag or on the plastic material or on the cellulose based material.

A system comprising an extracorporeal blood treatment device with a substitution pump (e.g. for pre- or post-substitution), and the reinfusion tube system, wherein the pump-loop section is positioned in a pump bed of the substitution pump may be provided. The extracorporeal blood treatment device may be a dialysis, hemodialysis, hemofiltration, hemodiafiltration, ultrafiltration, or plasmapherese device. The extracorporeal blood treatment device may also allow for one or more of the treatment methods.

In some embodiments the method according to the present invention may further encompass the step of connecting the check valve adapter to the post-loop section after having primed the arterial blood line via the reinfusion fluid tubing.

In several embodiments the method according to the present invention may further encompass the step of priming the arterial blood line via the reinfusion tube system.

The reinfusion tube system may be used for blood reinfusion only or for priming and reinfusion of blood. In particular, the check valve may only be used for reinfusion but may be omitted for priming. The reinfusion tube system may also be used during a treatment to supply liquid to an intermittent section of an extracorporeal blood tubing system with or without the check valve, in particular, when the check valve is removably attached to a tube of the reinfusion tube system. Intermittent has the meaning of a position along a blood tubing system that is not an end of the blood tubing system that is connected to the patient or the dialyzer during treatment.

In some embodiments the first tubular element and/or the second tubular element are integrally formed, cannot be released from each other, are comprised within one shared housing, form one non-elastic piece and/or are embodied as line connectors, in particular as Luer lock connectors.

In some embodiments the check valve adapter is not longer than 10 cm, preferably less than 7 cm, more preferably less than 5 cm long.

In some embodiments the reinfusion tube system comprises, at least at one end of its perfusable lumen, a line connector, in particular a female or male Luer connector.

In several embodiments, the reinfusion tube system is not entirely or partially part of a blood treatment cassette.

In some embodiments, the reinfusion fluid line is, or comprises, no substituate line and/or is not used for substitution. In several embodiments, it is used in addition to a substituate line.

In several embodiments, the reinfusion tube system comprises no patient needle, no line connector for directly connecting the reinfusion fluid line to an arterial or venous blood chamber of an extracorporeal blood circuit, no sensor, in particular no pressure sensor, and/or no connecting point, provided for connecting the reinfusion fluid line to such a sensor.

In some embodiments, the reinfusion fluid tubing and its connectors attached thereto, if present, may have, except for the check valve, no further flow blocking element. "Flow blocking element" means any element that hinders a flow out of the tubing system. The flow blocking element may act on or within the reinfusion fuid tubing without any external force to block the flow. Such a commonly known flow blocking element may be for example a septum. With the reinfusion fluid tubing according to the present invention, with connectors attached thereto, such a flow blocking element, for example the above-mentioned septum, provided in the port connector configured for connecting the reinfusion tube system to the extracorporeal blood treament device, in particular to the reinfusion liquid port, may be omitted.

In several embodiments, the reinfusion port may be used as substitution port during the treatment. Preferably, the control unit of the extracorporeal blood treatment device is configured to submit a substituate, or substitution fluid, during treatment of a patient (this step not being encompassed by the present invention) via said reinfusion port.

In some embodiments, the reinfusion port may be used as priming port during filling of the extracorporeal blood tubing system. Preferably, the control unit of the extracorporeal blood treatment device is configured to submit a priming fluid before treatment of a patient via said reinfusion port.

One or several of the above or in the following mentioned advantages may be achieved by some embodiments according to the present invention.

In the known use of reinfusion fluid tubings which are placed in or put into an occluding pump, a backflow of blood or blood particles, i.e. a retrograde flow of blood from the arterial line connected to the reinfusion fluid tubing into the hydraulics likewise connected - directly or indirectly, for example via connectors - to the reinfusion fluid tubing, is prevented by the occluding pump, e.g. the roller pump or the finger pump, when the pump is working as intended. However, this requires that the pump (e.g. its rotor-stator distance) and the hose diameter match one another. For this reason the manufacturer of the device undertakes intensive studies for ensuring the proper fit of the pump properties with the tubing properties. If a too thin or too thick hose as potentially being available on the market from differing tube system suppliers were used in the reinfusion, a backflow of blood particles into the hydraulics of the extracorporeal device may no longer be excluded. Similarly, it is required that the occluding force matches the stiffness of the hose for reliable occlusion by the rollers of the pump. For this reason, utmost care should be taken in practice that the hose size or dimensions and the pump type match each other. Of course, this also entails an increased effort in ordering, storing or generally keeping or holding reinfusion fluid lines having suitable diameters. Therefore, the check valve which may ensure that a backflow of blood through the reinfusion fluid tubing is excluded also reduces the risk of using reinfusion fluid tubings which do not optimally fit the present occluding pump with regard to material selection, flexibility and/or diameter. Potentially, a one-size-fits-all reinfusion fluid tubing which may be used in different but similar pumps, allows to deviations from the previous procedure due to the check valve of the reinfusion tube system according to the present invention. With the same level of hygiene achieved and thus safety for the patient, this may lead to significant simplification in the aforementioned logistics.

When mentioned herein that "with the protection" of the provided check valve also thin sections (thinner as required) of the reinfusion fluid tubing may be placed in the pump working in an occluding manner, this may also imply that the pump-loop section properties may be selected more independently from the safety aspect. Hence, it is possible through the present invention, for the first time to produce or make or manufacture the pump loop, supplemented by the check valve, in a more cost efficient manner. In this, if no particularly designed pump loop section is required, this may in turn help to save costs and effort during production. The check valve may offer this option.

After the disconnection of the patient, i.e. after having completed the treatment, the check valve in the blood reinfusion line also may advantageously prevent the spilling of particularly critical liquid out of the tube system, e.g. due to gravity, and consequently minimize the risk of contamination of the environment of the extracorporeal blood treatment device, the user and/or other patients. There are other means to avoid such a spilling known from prior art, for example manual clamps on the arterial and venous lines, but these are prone to human errors, for example, if the user forgets to close them.

Also, providing the check valve in the reinfusion line allows a higher degree of automatization by the possibility of removing the tube loop out of the reinfusion pump automatically, without any user interaction, e.g. clamping lines etc., after the reinfusion has been ended. The user then would only have to disconnect the venous line without further action.

### BRIEF DESCRIPTION OF THE DRAWINGS

The attached drawings and associated written description disclose exemplary embodiments of the present invention:
- FIG. 1: shows a high-level schematic drawing of a reinfusion tube system line with a check valve, the different letters show positions of the check valves according to different embodiments,
- FIG. 2: illustrates schematically some concrete examples of reinfusion tube system with check valve positions,
- FIG. 3: shows schematically embodiments of the check valve adapter having a check valve,
- FIG. 4: shows schematically embodiments of the reinfusion tube system package,
- FIG. 5: shows schematically an embodiment of a dialysis device with a mounted reinfusion tube system for supplying a liquid,
- FIG. 6: shows schematically an embodiment of the reinfusion tube system with the reinfusion fluid tube and the arterial and venous blood tubes and the dialyzer with a method for supplying liquid to the extracorporeal blood circuit, and
- FIG. 7: shows schematically embodiments of the method for reinfusion with a reinfusion tube system according to this invention.

### DETAILED DESCRIPTION

In the drawings, same or similar elements are denoted with the same reference numbers.

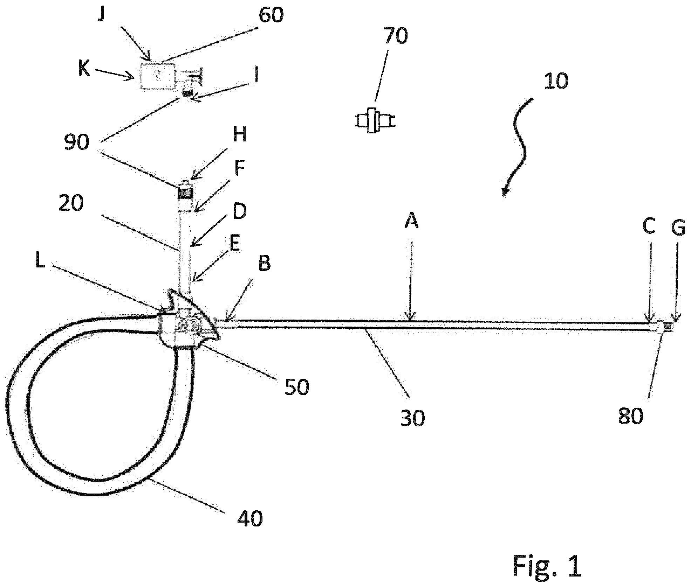

**FIG. 1** illustrates a schematic drawing of a reinfusion tube system 10. The reinfusion tube system 10 comprises a flexible fluid tubing with at least three sections, e. g., a pre-pump loop section 20, a post-pump loop section 30, and a pump loop section 40. The pump loop section 40 is positioned between the pre-pump loop section 20 and the post-pump loop section 30. The connection between both the pre-pump loop section 20 and the post-pump loop section 30 to the pump loop section 40, respectively, may be provided by a loop connector 50, which may be a hard plastic part to which the ends of the tube sections are attached, for example by gluing or by mechanical fixing.

In particular, by connecting two ends of the pump loop section 40 to the loop connector 50, a loop may be formed. The reinfusion tube system 10 may further comprise a port connector 60 that may be firmly fixed or removably attachable to the pre-pump loop section 20. The port connector 60 may serve as a connector with a lumen to guide fluid out of an extracorporeal blood treatment device into the reinfusion tube system 10.

Further, the reinfusion tube system 10 includes a check valve 70. The check valve 70 may be positioned anywhere along the reinfusion tube system 10. The check valve 70 blocks fluid flow from entering the reinfusion tube system 10 at the post-pump loop section 30 and exiting the reinfusion tube system 10 at the pre-pump loop section 20 or the port connector 60. This blocking function allows for blocking a flow into the reinfusion tube system 10 at the post-pump loop section 30 and out of the reinfusion tube system 10 at the pre-pump loop section 20 or the port connector 60. With such an orientation liquid may be supplied from the extracorporeal blood treatment device towards a line that has been connected to the post-pump loop section 30 but flow in the opposite direction is blocked. This also includes the situation that fluid may enter the reinfusion tube system 10, for example at the post-pump loop section 30, but not to exit the pre-pump loop section 20. Thus, it is to be understood that the check valve 70 may also be positioned at one of the ends of the reinfusion tube system 10. This positioning at the ends of the reinfusion tube system may be realized in embodiments of the reinfusion tube system using a connector, for example the check valve adapter I, II, III or a connector (not shown) changing the gender of the line connector 80 at the end of the post-pump loop section 30 from a male Luer lock connection to a female Luer lock connection.

In another embodiment, the check valve 70 may be integrated into the loop connector 50.

**FIG. 1** schematically shows possible positions for the check valve 70 with respect to the elements of the reinfusion tube system 10. The position of the check valve 70 may be any one of the group of positions including a position A which corresponds to a position in the post-pump loop section 30, a position B which corresponds to a position between the post-pump loop section 30 and the loop connector 50, a position C which corresponds to a position in the post-pump loop section 30 at a downstream end of the post-pump loop section 30, a position D which corresponds to a position in the pre-pump loop section 20, a position E which corresponds to a position between the pre-pump loop section 20 and the loop connector 50, a position F which corresponds to a position in the pre-pump loop section 20 at an upstream end of the pre-pump loop section 20, a position G which corresponds to a position at a downstream end of the post-pump loop section 30, a position H which corresponds to a position at an upstream end of the pre-pump loop section 20, a position I which corresponds to a position at a downstream end of the port connector 60, a position J which corresponds to a position within the port connector 60, a position K which corresponds to a position at an upstream end of the port connector 60, and a position L which corresponds to a position integrated into the loop connector 50.

The reinfusion tube system 10, may be fully, partially modular or a non-modular system.

In a non-modular system, elements of the reinfusion tube system 10 that are used to reinfuse the blood are non-removably attached to or integral with each other, and only connectors, such as the port connector 60 and the line connector 80 used to connect the reinfusion tube system 10 to the blood line system may be provided. In such a system, the check valve 70 is firmly fixed in its position within the reinfusion tube system 10 as part of the reinfusion tube system 10.

In a partially modular system, some elements are or may be removably attached to other elements of the system using specific connectors while other elements are firmly fixed to each other. Such a firmly fixed connector may be a line connector 90 that may be used to connect the pre-pump loop sections 20 to the port connector 60. Also, the check valve 70 may be provided as a removably attachable element as part of the reinfusion tube system 10. The pump loop section 40 may be non-removably fixed, for example, glued, to the pump loop connector 50. Also, the pre-pump loop section 20 and/or the post-pump loop section 30 may be non-removably fixed to the pump loop connector 50.

In the fully modular system, all elements may be removably or releasably fixed to each other.

The removable or releasable connections between the different elements may be provided, e.g., by Luer connectors and/or other key-lock types of connectors.

The reinfusion tube system 10 may also comprise one or more, for example, two, three or more check valves in any one of the positions A, B, C, D, E, F, G, H, I, J, K, L.

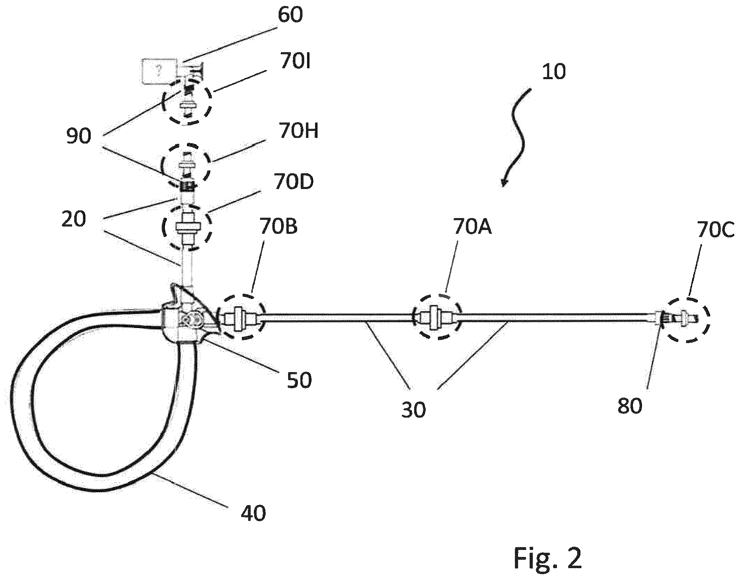

In **FIG. 2**, some embodiments of the pump tube system 10 described with respect to FIG. 1 are schematically shown. The check valve 70 is described in its potential positions as 70A to 701. The check valve 70A is positioned in the post-pump loop section 30. The check valve 70A is integrated, either directly or via a separate housing into the post-pump loop section 30. The check valves 70B and 70C are positioned at two opposite ends of the post-pump loop section 30, respectively. In some embodiments, the check valve 70C may be connected via a Luer (male-female) connection to the post-pump loop section 30, wherein one of the Luer connectors is connected to the post-pump loop section 30 while the other Luer connector is part of the separate housing in which the check valve is comprised. The separate housing may have a female Luer connector. In a similar manner to check valve 70A the check valve 70D is positioned in the pre-pump loop section 20 and the check valve 70H is positioned at the upstream end of the pre-pump loop section 20. Also comparable to the check valve 70C, the check valve 70H may be connected via a Luer connector to the pre-pump loop section 20. The check valve 701 is connected to the port connector 60. It is noted, that the check valve 70H and 701, in a fully assembled status, may correspond to each other with respect to their respective position and may differ basically only before the reinfusion tube system is assembled.

It is noted that in FIG. 2 not all possible positions are explicitly illustrated. Rather, with respect to possible positions it is referred FIG. 1.

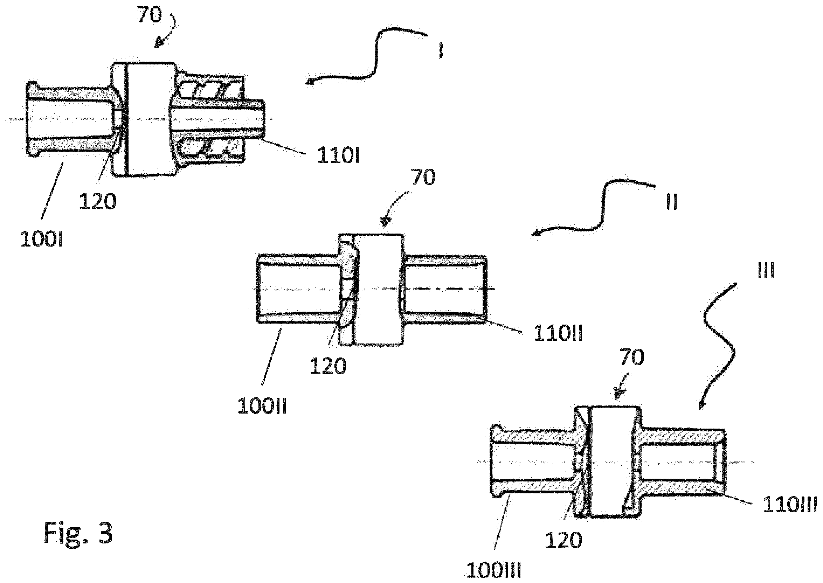

**FIG. 3** schematically shows three exemplary embodiments I, II, and III of the check valve adapter, each comprising a check valve 70. Check valves are known in the art, for example, they are described in US 6,390,120 B1, the entire, respective disclosure of which is incorporated by reference into this application.

The check valve adapters I, II, III may each comprise a first tubular element 100I, 100II, and 100III and a second tubular element 110I, 110II, and 110III that respectively define an upstream and a downstream passageway.

Each first tubular element 100I, 100II, and 100III may optionally be , coaxial to its corresponding second tubular element 110I, 110II, and 110III, respectively.

Each first tubular element 100I, 100II, and 100III and each second tubular element 110I, 110II, and 110III may be embodied as a connector for connecting with the end of a tubing section, in particular the pre-pump loop section 20, the post-pump section 30 or the pump loop section 40.

In any of the first tubular elements or in any of the second elements, or between each first tubular element 100I, 100II, and 100III and its corresponding second tubular element 110I, 110II, and 110III, repectively, the check valve may be positioned. In the example of FIG. 3, the check valve is embodied as a diaphragm 120 of elastically deformable material. The diaphragm 120 may be transversely positioned, sealingly cooperating with an optional annular valve seat of the said first tubular element 100I, 100II, and 100III or second tubular element 110I, 110II, and 110III, to form a fluid seal that maintains the check valve 70 in a normally closed position, and in which a predetermined fluid pressure in the said upstream passageway causes a deflection of the diaphragm 120 and consequently or accompanying an opening of the check valve 70.

It goes without saying that in FIG. 3 the check valve 70 is exemplarily arranged to block fluid flow from the second tubular element 110I, 110II, and 110III into the first tubular element 100I, 100II, and 100III.

Alternative embodiments of the check valve 70 may encompass as movable element a ball or a piston or any other element, wherein the movable element is configured to close the flow-through opening in the blocking direction.

The different embodiments I, II, III feature different combinations of the first and second tubular elements to generate fluid connections. It is understood that tubular elements of different types or embodiments may be combined in any combination as the connecting tubing or fluid lines they are connected with are independent from each other.

In the embodiment I, the first tubular element 100I is a female Luer lock and the second tubular element 110I is a male Luer lock. In the embodiment II, the first tubular element 100II is a hose to which, either on the inside or on the outside, a tube may be attached, for example by gluing or by mechanical fixing such as a friction, and the second tubular element 110II is a hose to which, either on the inside or on the outside, a tube may be attached, for example by gluing or mechanical fixation such as friction or clamping. In the embodiment III, the first tubular element 100III is a female Luer lock and the second tubular element 110III is also a female Luer lock.

The Luer lock connections may be preferably be used for removable or releasable connections, whereas the simple hose structure is preferred for non-removable or non-releasable connections.

The material of the check valve adapter I, II, III or any other check valve housing may be, e. g., any hard plastic or resin such as polycarbonate. Also, the deformable material of the diaphragm 120 may be any flexible polymer, in particular, silicone.

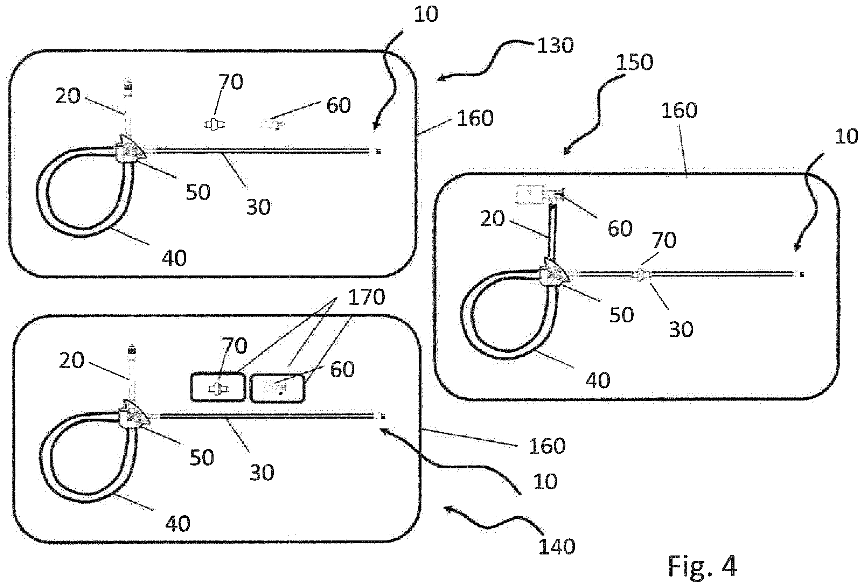

**FIG. 4** schematically illustrates three embodiments of the reinfusion tube system package according to the present invention, denoted by reference numerals 130, 140 and 150, repectively.

In each embodiment shown in FIG. 4 the reinfusion tube system package 130, 140 or 150 comprises a system container 160 encompassing the reinfusion tube system 10 according to the present invention, preferably under sterile conditions. Alternatively, the reinfusion tube system package 130, 140 or 150 may consist of the system container 160 and its reinfusion tube system 10.

The system container 160 may be flexible and may be fully or partially transparent.

The system container 160 may comprise or be made of a plastic or coated paper or both, for example the plastic forming a first side and the coated paper forming a second side.

The system container 160 may be sterilized internally or at the inside thereof.

The reinfusion tube system 10 may be provided as two, three or more physically separate elements. This is schematically shown in the exemplary package 130 wherein the sections 20, 30 and 40 are connected to each other via the loop connector 50, whereas the check valve 70 is provided as a separate component within the system container 160 as is the port connector 60.

In another embodiment, the package 140 corresponds to the package 130 with the difference that the check valve 70 is provided within the system container 160 inside a separate element container 170. The same may apply to the port connector 60.

It is noted that in any one of the embodiments the element container 170 may comprise only one item and/or only one check valve 70 or only one check valve adapter I, II, III.

What has been stated for the system container 160 herein may hold true for the element container(s) 170 as well, in particular with respect to sterility, transparency, material and the like.

In yet another embodiment of the package 150, all elements are pre-assembled and provided together as one physical system or unit in the system container 160.

In any embodiment, another tubing or further tubing lines such as an arterial blood tube and/or a venous blood tube and/or additional accessories may be comprised within the system container 160.

Hence, in the package 130 the system container 160 comprises both the reinfusion fluid tubing and the check valve 70 (which can be provided as part of a check valve adapter) (and optionally also the port connector 60), the reinfusion fluid tubing still being separate from the check valve 70 (and also from the port connector 60). For using the reinfusion tube system 10, the check valve 70 (and also the port connector 60) have to be connected to the reinfusion fluid tubing before reinfusing blood. Providing them separately from each other may allow use of the reinfusion fluid tubing also during the priming step during which a check valve is not required.

As in the package 130, in the package 140 the reinfusion fluid tubing and the check valve 70 (and optionally also the port connector 60) are separate from each other. In this embodiment, they are separately comprised in separate containers.

In the package 150, however, the components comprised in the package are pre-connected to each other which may contribute to avoiding hygienic risk.

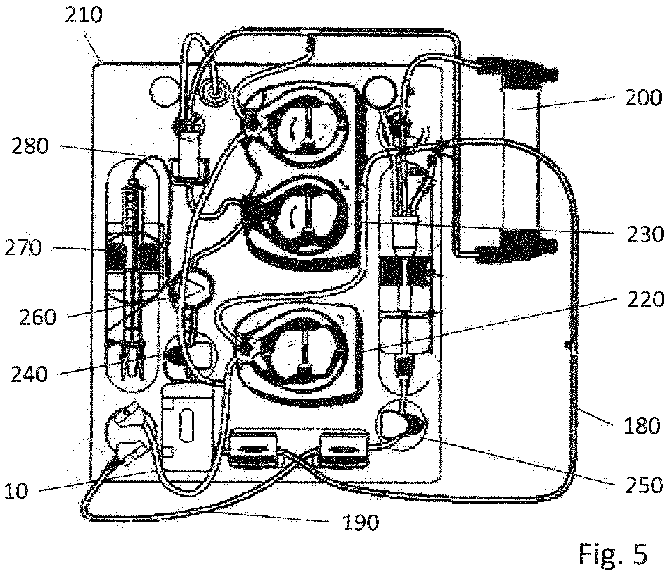

In **FIG. 5**, an exemplary embodiment of the extracorporeal blood treatment device 210 with a mounted or set-up reinfusion tube system 10, an arterial line 180, a venous line 190, and a dialyzer 200 is illustrated.

The extracorporeal blood treatment device 210 may further comprise one or more of the following elements: a priming and/or reinfusion pump 220 to position the pump loop section 40 of the reinfusion tube system 10 and to pump liquid from the liquid port through the reinfusion tube system 10, a blood pump 230 to pump blood through the arterial line 180, an arterial clamp 240 and a venous clamp 250 to block the flow in the arterial line 180 and the venous line 190, respectively, an arterial pressure sensor 260, a Heparin pump 270 that may be used to pump Heparin from an Heparin source to the arterial line 180 via a tubing line 280. The arterial pressure sensor 260 may be in a separate housing connected to the arterial line 180, the housing may have a circular base body, an inlet and an outlet, one side of the housing being optionally open and optionally covered by a flexible membrane so that the pressure prevailing within the housing has an effect on the form of the membrane.

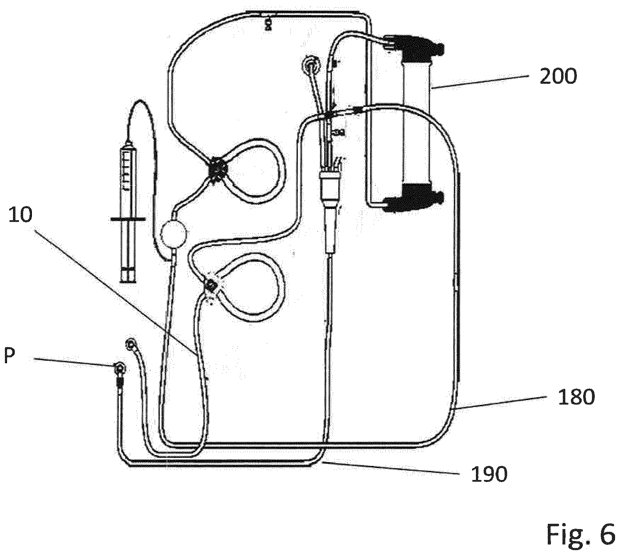

**FIG. 6** schematically shows the tubing of FIG. 5 in a connected state including the reinfusion tube system 10 in combination with an extracorporeal blood system comprising an arterial line 180, a venous line 190 and a dialyzer 200.

The arrangement illustrated in FIG. 6 is prepared or suitable for blood reinfusion as for the blood reinfusion process the venous line 190 is still connected to the patient's blood system. Hence, when liquid is entered along the reinfusion tube system 10 and into the arterial line 180, the blood is pushed along the arterial line 180 and back into the patient P.

A check valve (not shown in FIG. 6 and, hence, not denoted) in, or attached to, the reinfusion fluid tubing of the reinfusion tube system 10 reduces the risk that blood or other potentially problematic liquids can move, in a retrograde direction, from the blood tubing lines 180, 190 along the sections of the reinfusion fluid tubing and flow into the dialysis device's fluid circuit. The position of the check valve may be any position, in particular as described when referring to FIGS. 1 and 2.

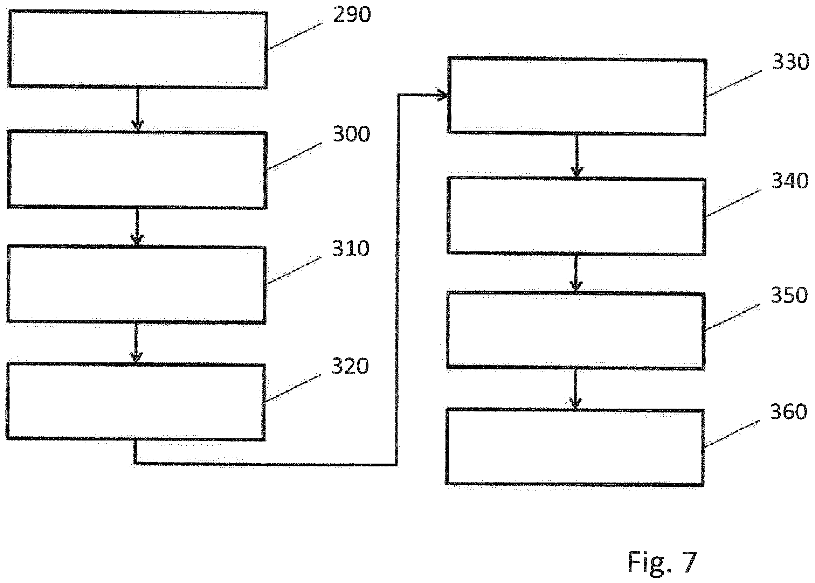

**FIG. 7** illustrates an embodiment of the method for reinfusion.

Before the reinfusion is started, the followings steps may be conducted, wherein the sequence of the steps shall not be defined by the listing below unless explicitly stated. For example, the separate elements may be attached to the extracorporeal blood treatment device 210 first and they may then be connected, or they may be first connected and then attached, or one element may be attached first before connecting and another one may be connected first before attaching:
In step 290, the tubing and the dialyzer 200 may be attached to the extracorporeal blood treatment device 210, meaning they are being brought in a functional position in which they are used during the following processes. For example, the reinfusion fluid tubing is positioned with its pump-loop section 40 in contact with a rotor of a pump of the extracorporeal blood treatment device 210. The arterial blood line 180 may be positioned with a blood pump-loop thereof in contact with a rotor of a blood pump 230 and in contact with the arterial pressure sensor 260 housed in an arterial pressure sensor housing.

In a step 300, the tubing is connected. This means the reinfusion fluid tubing is connected with a first end to a supply port of the extracorporeal blood treatment device 210, which may be a dialysis device, and with the second end to an end of the arterial blood line 180, either directly or via a check valve adapter I, II, III, wherein the check valve adapter I, II, III may comprise the check valve 70.

The second end of the arterial line 180 is connected to a port of the dialyzer 200, in particular the blood chamber of the dialyzer 200, whereas another port, in particular another port of the blood chamber of the dialyzer 200, is connected to an end of the venous blood line 190, and the second end of the venous blood line 190 is connected to a drain port of the extracorporeal blood treatment device 210, here: dialysis device, the drain port being part of a fluid circuit for guiding the fluid towards a drain.

In step 310, the extracorporeal blood circuit including the dialyzer 200 may be filled or primed with fluid via the reinfusion tube system 10.

In step 320, the reinfusion tube system 10 is disconnected from the end of the arterial blood line 180, and the venous blood line 190 is disconnected from the drain port. The arterial end and the venous end which have been disconnected from each other are connected to the blood circuit of the patient, e. g., via needles.

In step 330, the treatment is conducted by conveying liquid through the extracorporeal blood circuit.

After the treatment, in step 340, the arterial blood line 180 is being disconnected from the patient's blood circuit and is being connected to the reinfusion fluid tubing. If the check valve 70 is integrated into a separate transfer piece or connector element such as the check valve adapter I, II, III, this component must be used when the fluid connection from the supply port to the arterial blood line 180 via the reinfusion fluid tubing is established, either between the supply port and the reinfusion fluid tubing or between the reinfusion fluid tubing and the end of the arterial blood line 180.

In step 350, the liquid is being transferred from the supply port towards the arterial blood line 180 and the blood is being pushed by this liquid into the patient.

When the reinfusion is finished, in step 360, the venous blood line 190 may be disconnected from the patient P.

**Reference Numerals**

| | |
|---|---|
| 10 | reinfusion tube system |
| 20 | pre-pump loop section |
| 30 | post-pump loop section |
| 40 | pump loop section |
| 50 | loop connector |
| 60 | port connector |
| 70 | check valve |
| 70A to 70I | check valves in particular positions |
| 80 | line connector |
| 90 | line connector |
| 100I, 100II, 100III | first tubular element |
| 110I, 110II, 110III | second tubular element |
| 120 | diaphragm |
| 130 | reinfusion tube system package |
| 140 | reinfusion tube system package |
| 150 | reinfusion tube system package |
| 160 | system container |
| 170 | element container |
| 180 | arterial blood line |
| 190 | venous blood line |
| 200 | dialyzer |
| 210 | extracorporeal blood treatment device |
| 220 | priming or reinfusion pump |
| 230 | blood pump |
| 240 | arterial clamp |
| 250 | venous clamp |
| 260 | arterial pressure sensor |
| 270 | Heparin pump |
| 280 | tubing line |
| 290 | step |
| 300 | step |
| 310 | step |
| 320 | step |
| 330 | step |
| 340 | step |
| 350 | step |
| 360 | final step |
| A to L | position of check valve |
| I, II, III | check valve adapter with check valve |
| P | patient |

## Claims

1. A reinfusion tube system (10) comprising
- a reinfusion fluid tubing comprising a pre-pump loop section (20), a post-pump loop section (30), and a pump loop section (40); and either
- a check valve (70) positioned at an end or between the ends of the reinfusion fluid tubing such that it is provided to block fluid flow through the check valve (70) in a direction from the post-pump loop section (30) to the pre-pump loop section (20), or
- a check valve adapter (I, II, III) comprising a check valve (70), the check valve adapter (I, II, III) being configured to be connected to an end or to be positioned between the ends of the reinfusion fluid tubing such that the check valve (70) is provided to block fluid flow through the check valve (70) in a direction from the post-pump loop section (30) to the pre-pump loop section (20).

2. The reinfusion tube system (10) according to claim 1, wherein an end of the reinfusion fluid tubing or of the check valve adapter (I, II, III) comprises a female Luer lock connector configured for a connection with a male Luer lock connector arranged, e.g., at an end of an arterial blood line (180).

3. The reinfusion tube system (10) according to any one of the preceding claims, wherein the check valve adapter (I, II, III) is configured to be connected to an arterial blood line (180) with a first end for providing a fluid connection from the post-pump loop section (30) to the arterial line (180).

4. The reinfusion tube system (10) according to any one of the preceding claims, wherein the check valve (70) is, preferably arranged in a housing, irremovably attached or removably attachable to the pre-pump loop section (20), the post-pump loop section (30), the pump loop section (40) and/or the check valve adapter (I, II, III) or between any two of those.

5. The reinfusion tube system (10) according to any one of the preceding claims wherein the check valve (70) has a blocking characteristic such that:
in the closing direction the maximum backpressure at which the check valve still blocks a flow is 6.9 bar, 6 bar, 5 bar, 4 bar, 2.8 bar, 2.7 bar or 2.5 bar, respectively.

6. The reinfusion tube system (10) according to any one of the preceding claims wherein check valve (70) has an opening characteristic such that:
the minimum opening pressure is 200 mbar, 300 mbar, 400 mbar or 500 mbar, respectively.

7. The reinfusion tube system (10) according to any one of the preceding claims wherein the check valve (70) has a flow-passing characteristic such that:
a ratio between a minimum opening pressure and the maximum backpressure is or is below 0.2, 0.1, 0.05, wherein the opening pressure is the pressure difference across the check valve (70) in a flow direction from the pre-pump loop section (20) to the post-pump loop section (30) and the backpressure is the pressure difference across the check valve (70) in a flow direction from the post-pump loop section (30) to the pre-pump loop section (20).

8. A reinfusion tube system package (130, 140, 150) comprising
- a system container (160), in particular a bag or a blister pack;
- a reinfusion tube system (10) according to any one of the preceding claims;
wherein the reinfusion tube system (10) is comprised within the system container (170).

9. The reinfusion tube system package (130, 140, 150) according to claim 8, the package (130, 140, 150) further comprising
- an element container (170), in particular a bag or a blister pack;
wherein the check valve adapter (I, II, III) is comprised within the element container (170), and
wherein the element container (170) is comprised within the system container (160).

10. A system comprising
- an extracorporeal blood treatment device (210) with a priming or reinfusion pump (220), in particular a substitution pump, and
- the reinfusion tube system (10) according to any one of the claims 1 to 9,
wherein the pump-loop section (40) is positioned in a pump bed of the priming or reinfusion pump (220).

11. A method for reinfusing blood in a blood tubing set (10) encompassing the steps of
- connecting the reinfusion tube system (10) according to any one of claims 1 to 9 to both a reinfusion port of an extracorporeal blood treatment device (210) and to an arterial line (180); and
- supplying a reinfusion fluid from a reinfusion fluid source via the reinfusion port to the reinfusion tube system (10).

12. A method for preparing an extracorporeal blood treatment device (210) with a reinfusion fluid tubing before reinfusing blood into a patient's vessel, the method encompassing the step
- connecting the reinfusion tube system (10) according to any one of claims 1 to 9 to a reinfusion port of the extracorporeal blood treatment device (210), such that the check valve (70) is positioned or orientated so as to block fluid flow through the check valve (70) in a direction from the post-pump loop section (30) to the pre-pump loop section (20) and/or towards the extracorporeal blood treatment device (210);
- or connecting the check valve adapter (I, II, III) of the reinfusion tube system (10) to the reinfusion fluid tubing, in particular to the post-pump loop section (30).

13. The method according to claim 12, further encompassing the step of
- connecting the check valve adapter (I, II, III) to the post-loop section (30), after having primed the arterial blood line (180) via the reinfusion fluid tubing.

14. The method according to claim 12 or claim 13, further encompassing the step of
- priming the arterial blood line (180) via the reinfusion tube system (10).

15. A check valve adapter (I, II, III) comprising, or consisting of, a first tubular element (100I, 100II, 100III), a second tubular element (110I, 110II, 110III) in fluid communication with the first tubular element (100I, 100II, 100III) and a check valve (70) arranged in the fluid communication between first and second tubular element.
